# EUROPEAN PATENT APPLICATION

(11) **EP 4 130 295 A1**
(43) Date of publication of application: **08.02.2023**
(21) Application number: 21382731.4
(22) Date of filing: 03.08.2021
(51) Int. Cl.: C12Q 1/6883

(54) **INFORMATIVE BIOMARKERS OF PORTAL HYPERTENSION**

(71) Applicant: Institut D'Investigacions Biomèdiques August Pi I Sunyer - IDIBAPS, 08036 Barcelona (ES); Hospital Clínic de Barcelona, 08036 Barcelona (ES); Consorcio Centro de Investigación Biomédica en Red M.P., 28029 Madrid (ES)
(72) Inventor: GRACIA SANCHO, Jordi, 08036 BARCELONA (ES); ORTEGA RIBERA, Martí, 08036 BARCELONA (ES); GIBERT RAMOS, Albert, 08036 BARCELONA (ES); GARCIA PAGÁN, Joan Carles, 08036 BARCELONA (ES); MAGAZ MARTÍNEZ, Marta, 08036 BARCELONA (ES)
(74) Representative: ZBM Patents - Zea, Barlocci & Markvardsen

(57) **Abstract**

The present inventors provide non-invasive biomarkers (E-cadherin (ECAD) and/or SPINK1) for the diagnosis and prognosis of portal hypertension.

Advantageously, the markers of the invention also allow to appropriately differentiate between the non-clinical and clinically significant portal hypertension, thus being possible a more precise management of the therapeutic protocols.

## Description

### Technical Field

The present invention is related to the field of diagnosis. In particular, the present invention provides non-invasive methods for the diagnosis of portal hypertension, based on measuring the amount of circulating CBX7-regulating proteins, such as E-cadherin (ECAD) and/or SPINK1 in a bodily fluid sample.

### Background Art

Advanced chronic liver disease (aCLD) is nowadays the 11th most common cause of death globally, with approximately 1.16 million deaths per year. Alcohol abuse, chronic viral hepatitis B or C infection and metabolic-associated fatty liver disease are amongst the most frequent causes of this condition.

Portal hypertension (PH) is one of the main drivers of aCLD-related clinical complications including bleeding from gastro-esophageal varices, ascites, hepatorenal syndrome or hepatic encephalopathy among others, and predisposes the cirrhotic patient to acute-on-chronic liver failure.

PH can be defined as an abnormally increase portal venous pressure - blood pressure in the portal vein and its branches, that drain from most of the intestine to the liver. Portal hypertension is defined as a hepatic venous pressure gradient equal or greater than 5 mmHg.

PH is usually studied as a consequence of the complex cirrhosis syndrome, being accompanied by other confounder factors such as increased stiffness, altered shear stress or immune infiltrate.

Currently, hepatic venous pressure gradient (HVPG) is the most reliable method for assessing PH and allows stratification of patients with normal pressure (NP, HVPG ≤ 5mmHg), PH (HVPG > 5mmHg) and clinically significant PH (CSPH, HVPG ≥10mmHg). In patients with more than 10 mmHg and especially above 12mmHg, clinical complications appear, being at higher risk of hepatic decompensation and death. However, HVPG measurement is an invasive procedure with some limitations including availability and affordability in all centres, requirement of high-technical skills, patient discomfort and improbable but existing risk of severe complications such as bleeding. Recently, transient elastography has been in the spotlight as an alternative technique for assessing PH and fibrosis. Still, inter-professional interpretation of the data, lobule bias and high costs for primary attention centres represent the main hindrances in the appropriate diagnostic and monitoring of PH.

Therefore, there is still the need of ubiquitous and reliable non-invasive tests for identifying and precisely stratifying patients suffering from PH.

### Summary of Invention

The present inventors have found that pathological hydrodynamic pressure induces LSEC disfunction. In particular, they have found that chromobox 7 (CBX7), mainly expressed in LSECs, was a pressure-related factor.

As it is shown below, the inventors have surprisingly found that CBX7 downregulation correlates with PH. Tables 3 and 4 below provides the CBX7 liver expression in the discovery and validation cohorts: these data show that CBX7 exhibits an excellent diagnostic ability, being above 0.965 with a sensitivity of 100% and a specificity of at least 75% and 90%, respectively.

The inventors further found that CBX7-regulated circulating proteins such as E-cadherin (ECAD) and serine peptidase inhibitor, Kazal type I (SPINK1), were differentially expressed in plasma samples from healthy subjects and subjects suffering PH (above 5 mmHg). Table 5 shows that both markers are upregulated when the subject suffers from PH.

Not only that, but also, the present inventors have found that these CBX7-regulated circulating proteins are also differentially expressed in CSPH (See Table 6 below).

Altogether, both ECAD and SPINK1, are non-invasive markers which provides robust and precisely identification of a patient suffering from PH.

Thus, in a first aspect the present invention provides a method of diagnosis portal hypertension (PH) in a subject, the method comprising the steps of: (a) measuring the amount of the gene expression product of one or both of the markers E-cadherin (ECAD) and SPINK1 in an isolated fluid sample from the subject; (b) comparing the amount with a reference value; and (c) diagnosing the portal hypertension when the measured amount is higher than the reference value.

As stated above, HVPG is the standard technique for assessing PH but, despite being the most trustable procedure, it has some limitations such as universal coverage, need for skilful professionals, patient discomfort and risk of complications.

The present invention means a great advance in the diagnosis and appropriate monitoring of the disease: two markers, which can be easily quantified in a fluid sample, provide a robust information to classify a subject as suffering or not PH.

Not only that, but also, the present inventors have also found that these markers provide information of the severity of the disease: the highest the amount of the marker is, the highest is the pressure within the portal vein, which will indicate that the subject is in advance phase of the PV, suffering a clinically significant PH.

In view of the above, the ECAD and SPINK1, not only provide a non-invasive method for identifying subjects suffering PH (when they are overexpressed), but also they are so "sensitive" to the pressure within the portal vein, that can precisely inform of the real situation of subject, depending on the amount measured. Therefore, these markers provide robust diagnostic and monitoring information to the physician: the highest amount, the highest the pressure within the portosinusoidal system.

Thus, in a further aspect the present invention provides a method of prognosis PH, the method comprising measuring the amount of the gene expression product of one or both of the markers ECAD and SPINK1 in an isolated fluid sample from the subject, at least at two different temporal points; wherein if there is an increase in the amount of expression product, this indicates that there is a bad prognosis, particularly this is indicative that the subject is at risk of progressing to severe portal hypertension or decompensation.

In a further aspect the present invention further provides a method for stratifying a subject as normal portal pressure (NP), as suffering from non-clinically significant PH (NCSPH) or as suffering clinically significant PH (CSPH), the method comprising the steps of:
(i) measuring the amount of expression gene product of one or both of the markers selected from ECAD and SPINK1 in an isolated fluid test sample of the subject;
(ii) obtaining reference control values V1 and V2, wherein:
   V1 is calculated measuring the amount of the marker(s) in a group of samples from subjects with a portal pressure equal or lower than 5 mmHg; and
   V2 is calculated measuring the amount of the marker in a group of samples from subjects with a portal pressure equal or lower than 10 mmHg, and higher than 5 mmHg;
(iii) comparing the measured amount of step (i) with the reference control values V1 and V2, wherein:
   if the measured value of (i) is equal or below V1, the subject is identified as "normal portal pressure";
   if the measured value of (i) is between V1 and V2, the subject is identified as suffering NCSPH; and
   if the measured value of (i) is equal or above V2, then the subject is identified as suffering CSPH.

The plasmatic factors ECAD and SPINK1 allow the correct detection and classification of patients with NCSPH or CSPH in a routine blood test. This novel analytical approach could be easily implemented into decision making for patients with chronic liver disease (CLD), for instance, who may avoid unnecessary HVPG measurement if the plasmatic values for the ECAD and/or SPINK1 is above the proposed cut-off values. But also, provides therapeutic advantages, in the managing of the most appropriate therapeutic approach.

Therefore, in a further aspect, the present invention provides a method of deciding or recommending whether to initiate a medical regimen of a subject suspicious of suffering PH (either as "NCSPH" or "CSPH"), which method comprises the steps of:
Step a:
   a.1) diagnosing the subject as suffering; or, alternatively,
   a.2) prognosing the subject; or, alternatively,
   a.3) stratifying the subject as NCSPH or CSPH,
   following any of the above provided methods of the invention, and
Step b: deciding or recommending to initiate the medical regimen if the subject is diagnosed as suffering PH or stratified as NCSPH or CSPH.

The medical regimen in case of portal hypertension involves lactulose, enemas, and use of antibiotics such as rifaximin, neomycin, vancomycin, and the quinolones. This regimen also considers in those more severe stages surgery interventions based, for instance, in portosystemic shunts, and fluoroscopic image of transjugular intrahepatic portosystemic shunt (TIPS). Diagnosis of patients with NCSPH, which is currently difficult, is highly relevant since these patients may be at the onset or initial stages of the disease. No specific treatment is prescribed to these individuals, besides removal of the etiological factor and lifestyle recommendations. In the case of patients with CSPH, non-selective beta-blockers is currently the gold-standard therapy to ameliorate portal hypertension. In patients with clinical complications such as ascites, peritonitis and hepatic encephalopathy additional treatments may be required (diuretics, antibiotics or dialysis).

In a further aspect, the present invention provides a method for determining the efficacy of a medical regimen in a patient already diagnosed of PH (either as "NCSPH" or "CSPH"), the method comprising the steps of:
(a) measuring the amount of the expression gene product of one or both of the markers ECAD and SPINK1 in an isolated fluid sample from the patient prior to the administration of the medical regimen;
(b) measuring the amount of the expression gene product of the one or both markers in an isolated fluid sample from the patient once started the administration of the medical regimen; and
(c) comparing the levels measured in steps (a) and (b), in such a way that if the amount measured in step (b) is lower than the amount measured in step (a), it is indicative that the medical regimen is effective in the treatment of the condition.

In a further aspect, the present invention provides the use of ECAD alone or in combination with SPINK1 in the diagnosis and prognosis of PH; in the stratification of a subject as normal portal pressure, as suffering from non-clinically significant PH (NCSPH) or as suffering clinically significant PH (CSPH); in a method of deciding or recommending whether to initiate a medical regimen of a subject suspicious of suffering PH; or for determining the efficacy of a medical regimen in a patient already diagnosed of PH.

In a further aspect, the present invention provides the use of means for performing any of the methods provided by the present invention.

In a further aspect, the present invention further provides a method of diagnosing, prognosing or stratifying a subject suffering from PH, the method comprising measuring the amount of CBX7 in a test sample of the subject.

And in a last aspect, the present invention provides the use of CBX7 for the diagnosis, prognosis or stratification of a subject suffering from PH.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides non-invasive methods for the diagnosis, monitoring and stratification of subjects suffering from PH, based on determining the amount of ECAD and SPINK1 in an isolated bodily fluid sample of the subject.

In the context of the present invention, "portal hypertension" (PH) is understood as a blood pressure in the portal vein and its branches, that drain from most of the intestine to the liver, equal or higher than 5 mmHg. PH can be measured using any commercial device. An illustrative example is the balloon-tipped catheter used in the Examples provided below, but the skilled person, using their general knowledge can use other alternative devices with the same end. The election of one or other device does not significantly affect to the pressure measure.

In the context of the present invention, "normal portal pressure" (NP) subject, means that the portal vein pressure is below 5 mmHg; "non-clinically significant PH" (NCSPH), means that the portal vein pressure is equal or higher than 5 but lower than 10 mmHg; and "clinically significant PH" (CSPH) means that the portal vein pressure is equal or above 10 mmHg.

E-cadherin (ECAD) is a cell adhesion molecule expressed by different cell types that can be found in the plasma as soluble e-cadherin, resulting from the proteolytic cleavage of the cell surface ECAD and indicating several processes including a disruption in cell-cell interaction and increases in cell migration and proliferation. SPINK1 has been reported to increase in the serum of different cancers and to be linked to the progression of HBV-related diseases.

The term "diagnosis" is known to the person skilled in the art. As used herein "diagnosis" is understood as becoming aware of a particular medical condition, syndrome, complication; the determination of the nature of the disease or condition; or the distinguishing of one disease or condition from another. It refers both to the process of attempting to determine or identify the possible disease or condition, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

"Prognosis" as used herein refers to the prediction of the probable progression and outcome of a disease.

The term "amount of expression product" as used herein have the same meaning as, and can be interchanged by, "level of expression product".

In the present invention, the term "reference value" referred in the methods of the invention has to be understood as a predefined value of a given molecular marker, which is derived from the levels of said molecular marker in a sample or group of samples. The samples are taken from a subject or group of subjects wherein the presence, absence, stage, or course of the disease has been properly performed previously. This value is used as a threshold to discriminate subjects wherein the condition to be analysed is present from those wherein such condition is absent, to determine the stage of the disease, or the risk of developing or of being suffering from a thrombosis condition, among others. This reference control level is also useful for determining whether the subject has to initiate a medical regimen.

Methods for obtaining the reference value from the group of subjects selected are well-known in the state of the art (Burtis C. A. et al., 2008, Chapter 14, section "Statistical Treatment of Reference Values"). In a particular case "reference value" is a cut-off value defined by means of a conventional ROC analysis. As the skilled person will appreciate, optimal cut-off value will be defined according to the particular applications of the diagnostic or prognostic method: purpose, target population for the diagnosis or prognosis, balance between specificity and sensibility, etc.

The subject or subjects from whom the "reference value" is derived may include subject/s wherein the condition is absent, subject/s wherein the condition is present, or both. The skilled person in the art, making use of the general knowledge, is able to choose the subject or group of subjects more adequate for obtaining the reference control level for each of the methods of the present invention. In an embodiment of the present invention, optionally in combination with any of the embodiments provided above or below, the reference value referred in the diagnostic method is taken from a group of subjects which do not suffered PH. The main criterium to consider the subject as not suffering PH is that the HVPG value is below 5 mmHg, when measured using any of the devices already available in the state of the art.

In the present invention, the term "HVPG" refers to the hepatic venous pressure gradient, and it is determined as the difference between the Wedged hepatic venous pressure in the main right or middel hepatic vein (WHVP) and the free hepatic venous pressure (FHPV). The venous pressure can be measured using any of the devices already available in the market, following manufacturer's instructions. For example, as shown below, the pressure can be measured using a 7F balloon-tipped catheter (such as "Fogarty" Edwards Lifesciences LLC, CA). All measurements can be taken by triplicate and averaged to obtain the baseline HVPG. Real-time measurements on hepatic venous pressure values were obtained in each case in a multichannel recorder (GE Healthcare, Milwaukee, WI).

In an alternative embodiment of the present invention, optionally in combination with any of the embodiments provided above or below, the reference value of the prognosis method corresponds to the measure of the marker(s) in a previous moment of time (in terms of days, weeks or months).

When combining ECAD and SPINK1, the better predictive power was obtained. ECAD + SPINK1 were able to discriminate patients with NP or PH with a sensitivity of 93.1% and specificity of 83.3%. But also, was able to discriminate patients CSPH vs NCSPH with a 91.4% sensitivity and 63.6% specificity.

Therefore, in one embodiment of the methods of the present invention, optionally in combination with any of the embodiments provided above or below, the amount of both, the ECAD and SPINK1 markers, is measured.

In another embodiment of the methods of the present invention, optionally in combination with any of the embodiments provided above or below, the fluid test sample is selected from the group consisting of whole blood, plasma, serum, urine and saliva. Particularly, the fluid sample is plasma, blood or serum.

In another embodiment of the methods of the present invention, optionally in combination with any of the embodiments provided above or below, the sample is isolated from a subject already diagnosed of suffering a liver disease, such as chronic liver disease, Budd-Chiari syndrome or portal vein thrombosis. The chronic liver disease can be in an earlier or advance stage. In one embodiment the chronic liver disease is chronic cirrhosis due to any aetiology including alcoholic abuse, viral hepatitis or metabolic liver disease.

Additionally, combining the expression of these CBX7-related targets with other scores or liver-derived parameters (i.e. platelet count, AST or bilirubin) will increase the prognostic value of the herein presented data.

Thus, in another embodiment of the methods of the present invention, optionally in combination with any of the embodiments provided above or below, the methods further comprise one or more of the following steps:
i) measuring the level of one or more of the following markers: cholesterol; inflammatory biomarkers such as IL-1β, IL-IRa, Fas-R, VCAM1 , TNF-β, HSP-70, IL-18, TLR9, lymphotoxin-β, glutamine, glutamine synthase, HSP-27, HSP-60, HSP-110, grpl70, hyaluronan, homeocysteine, or angiotensin- II; and /or
ii) further correlation with demographic and clinical laboratory parameters selected from the group consisting of age, model for end-stage liver diseases (MELD), Child-Pugh Score (CPS), platelets, alanine aminotransferase (ALT), aspartate aminotransferase (AST), platelet count, prothrombin time (PT/INR), liver stiffness, and at-risk alcohol use for indicating EGD to the patient or not indicating EGD for the patient.

Various mathematical approaches for correlating biomarker panels based on several markers and parameters, are well-known in the art. And mainly rely on statistical analysis known as multivariate classification or supervised learning. Exemplary methods, such as a Pearson's correlation coefficient, multiple linear regression analysis, threshold-based methods, logistic regression analysis, tree-based methods, and Support Vector Machine (SVM), can be applied to correlate biomarker panels with a clinical diagnosis, prognosis, stratification or indication for further treatment. Generalized additive models also allow one to combine data with patient clinical information to predict an outcome. Furthermore, several other methods, e.g., a Bayesian network on gene expression microarray data, perform well in proteomics based biomarker detection. Additional well-known data preprocessing and data normalization steps can be implemented. Additional computational methods and randomization techniques, such as a permutation test, cross-validation and bootstrapping, can help to evaluate and validate the performance and correlation. A more detailed discussion of methods for defining, classifying and performance validation of a biomarker panel are described in Robin *et al.,* 2009, the entire contents of which is incorporated by reference herein.

In another embodiment of the methods of the invention, optionally in combination with any of the embodiments provided above or below, the level of expression of the gene is determined by measuring or determining the amount of corresponding mRNA or protein (e.g., full-length protein product or a proteolytic fragment thereof), depending on the detection technique to be used.

In another embodiment of the methods provided by the present invention, the level of the protein markers or fragments thereof is determined by a quantitative test selected from the group consisting of an immunological test, bioluminescence, fluorescence, chemiluminescence, electrochemistry and mass spectrometry.

In one embodiment of the invention, the level of expression is determined by immunochemistry.

The term "immunochemistry" as used herein refers to a variety of techniques for detecting antigens (usually proteins and peptides, and in the present case any of the proteins listed above alone or in combination) in a sample by exploiting the principle of antibodies binding specifically to said antigens. Visualising an antibody-antigen interaction can be accomplished in a number of ways. In the most common instance, an antibody is conjugated to an enzyme, such as peroxidase, that can catalyse a colour-producing reaction. Alternatively, the antibody can also be tagged to a fluorophore, such as fluorescein or rhodamine. The immunochemistry technique can be direct or indirect. The direct method is a one-step staining method and involves a labelled antibody (e.g. FITC-conjugated antiserum) reacting directly with the antigen. While this technique utilizes only one antibody and therefore is simple and rapid, the sensitivity is lower due to little signal amplification, such as with indirect methods, and is less commonly used than indirect methods. The indirect method involves an unlabelled primary antibody (first layer) that binds to the target antigen in the sample and a labelled secondary antibody (second layer) that reacts with the primary antibody. This method is more sensitive than direct detection strategies because of signal amplification due to the binding of several secondary antibodies to each primary antibody if the secondary antibody is conjugated to the fluorescent or enzyme reporter.

Further amplification can be achieved if the secondary antibody is conjugated to several biotin molecules, which can recruit complexes of avidin-, streptavidin or Neutravidinenzyme. The indirect method, aside from its greater sensitivity, also has the advantage that only a relatively small number of standard conjugated (labelled) secondary antibodies needs to be generated. With the direct method, it would be necessary to label each primary antibody for every antigen of interest. It must be borne in mind that immunochemistry techniques can also be used to detect certain nucleic acid sequences if a tagged nucleic acid probe (designed to specifically bind to a certain target nucleic acid sequence) can later on be detected with a labelled antibody. Thus, the detection of the protein could be performed by using a tagged nucleic acid designed to bind a specific sequence of the target protein RNA, and then detecting said tagged nucleic acid with a labelled antibody which selectively binds to the tag.

Immunoassay procedures suitable include enzyme-linked immunosorbent assays (ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan.

In one embodiment, in combination with any of the embodiments provided above or below, the level of expression of protein(s) is determined by an immunoassay.

In another embodiment, in combination with any of the embodiments provided above or below, the level of expression of protein is determined by ELISA.

All the above embodiments, related to the methods of the invention, are also embodiments of the uses provided by the present invention, too.

In one aspect, the invention provides the use of means for performing any of the methods of the invention. In one embodiment, the means are antibodies or fragments thereof, which can form part of a kit. The kit may additionally comprise means (additives, solvents) to visualize the antibody-protein interactions.

The term "antibody or a fragment thereof able to bind to the target protein(s)" is to be understood as any immunoglobulin or fragment thereof able to selectively bind the target protein. It includes monoclonal and polyclonal antibodies. The term "fragment thereof encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The antibodies used for specific detection can be polyclonal or monoclonal. There are well known means in the state of the art for preparing and characterizing antibodies. Methods for generating polyclonal antibodies are well known in the prior art. Briefly, one prepares polyclonal antibodies by immunizing an animal with the protein; then, serum from the immunized animal is collected and the antibodies isolated. A wide range of animal species can be used for the production of the antiserum. Typically, the animal used for production of antisera can be a rabbit, mouse, rat, hamster, guinea pig or goat.

Moreover, monoclonal antibodies (MAbs) can be prepared using well-known techniques. Typically, the procedure involves immunizing a suitable animal with the protein associated with the disease. The immunizing composition can be administered in an amount effective to stimulate antibody producing cells. Methods for preparing monoclonal antibodies are initiated generally following the same lines as the polyclonal antibody preparation. The immunogen is injected into animals as antigen. The antigen may be mixed with adjuvants such as complete or incomplete Freund's adjuvant. At intervals of two weeks, approximately, the immunization is repeated with the same antigen.

The *in vitro* methods and uses of the invention provide an information in terms of diagnosis, prognosis, medical regimen and stratification. In one embodiment, the methods of the invention further comprise the steps of (1) collecting the information about the diagnostic, prognostic, medical regimen and/or stratification, and (2) saving the information in a data carrier.

In the sense of the invention a "data carrier" is to be understood as any means that contain meaningful information data for the diagnosis or prognosis of endometrial carcinoma, such as paper. The carrier may also be any entity or device capable of carrying the prognosis data. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the prognosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

Throughout the description and claims the word "comprise" and variations of the word are not intended to exclude other technical features, additives, components, or steps. Furthermore, the word "comprise" and its variations encompasses the term "consisting of. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Materials and Methods

### Animals

Male Wistar rats were kept at the University of Barcelona Faculty of Medicine facilities, housed three per cage, under controlled environmental conditions (19.7 ± 2°C, 52 ± 5% humidity, 12 hours light/dark cycle) and free access to standard rodent food pellets and water. All the experimental procedures were approved by the Laboratory Animal Care and Use Committee of the University of Barcelona and performed in accordance with the European Community guidelines for the protection of animals used for experimental and other scientific purposes (EEC Directive 86/609).

### Isolation of hepatic cells

Primary hepatocytes and non-parenchymal cells from healthy rats weighing 300-350 g were isolated using the 4 in 1 protocol as previously reported (Fernández-Iglesias, A. *et al*., 2019). Briefly, liver was perfused, digested with 0.015% collagenase A (103586, Roche, Sant Cugat del Vallès, Spain) and mechanically disaggregated obtaining a multicellular suspension. Hepatocytes were purified by low-speed centrifugation (5 min at 50g) and non-parenchymal cells were separated using a three-phase iodixanol (Optiprep^{™}, Sigma-Aldrich, Saint Louis, MO, USA) density gradient centrifugation. Afterwards, the upper interphase, that contained the hepatic stem cells (HSCs), was directly seeded in Iscove Modified Dulbecco Media (IMDM) culture media at 37°C 5% CO₂ in the incubator. The lower interphase, enriched in liver sinusoidal endothelial cells (LSECs) and Kupffer *cells* (KCs), was further purified by differential adherence time to non-coated substrates. After 30 min of culture of both cell types, KCs were attached to the culture plate while LSECs were found in the supernatant. Highly pure and viable LSECs cells (as described in Fernández-Iglesias A et al., 2019) were seeded on conventional culture plates or on liver-on-a-chip devices as described below

### Hydrodynamic pressure on a liver-on-a-chip

The study of hydrodynamic pressure *in vitro* was performed using an advanced sinusoidmimicking microfluidic device named as Exoliver. The details of its fabrication, features and suitability for sinusoidal studies have been previously reported by Ortega-Ribera, M. *et al.* 2018*.*

Primary cells isolated from healthy rats were exposed to physiological (1 mmHg) or pathological (12 mmHg) pressures within the liver-on-chip device for 48h. Laminar shear stress stimulus on LSECs cultures was maintained at 1.15 dyn/cm² (Ortega-Ribera, M. *et* al., 2019).

Desired pressure within the device was ensured by modulating outflow height relative to LSECs culture (Bernouilli's equation) and according to 1 mmHg = 1.36 cm H₂O. Real-time assessment of pressure within the bioreactor was routinely performed in independent experimental settings using a pressure probe both at the inflow and outflow of the device connected to a Powerlab (4SP). Data was displayed into a LabChart v5.5.6 software file.

Device configuration and culture was performed as previously described by Ortega-Ribera and colleagues (Ortega-Ribera, M. *et al.* 2019), with minor modifications. Briefly, LSECs were cultured on a hydrophilic biocompatible polytetrafluoroethylene microporous membrane (Omnipore, Millipore, USA), and hepatocytes, KCs and HSCs at a lower layer, immediately below and in contact with the endothelial fraction of the culture. Microfluidic cultures were maintained at 37°C and 5% CO₂, with 43 ml of a recirculating media comprising ²⁰; DMEM/F12 was supplemented with 2.97% dextran (31392; Sigma-Aldrich, Darmstadt, Germany), 2% fetal bovine serum (04-001-1A; Biological Industries, Kibbutz Beit-Haemek, Israel), 1% penicillin-streptomycin (10378-016, Biological Industries), 1% endothelial cell growth supplement (BT-203; Biomedical Technologies, Kandel, Germany), 1% heparin (H3393; Sigma-Aldrich), 1% L-glutamine (25030-024; Gibco, Dublin, Ireland), 1% amphotericin B (03-029-1C; Biological Industries), 1 nM dexamethasone (D4902; Sigma-Aldrich), 10 ng/ml Epidermal Growth Factor (E4127; Sigma-Aldrich), 1.5 nM glucagon (16941-32-4, Novo Nordisk, Plainsboro, NJ, USA), 15 nM hydrocortisone (H0888, Sigma-Aldrich), and 1 µM insulin (Humulin S, Lilly S.A.).

### mRNA sequencing

Primary LSECs transcriptome profile under physiological or pathological hydrodynamic pressures was examined by mRNA sequencing. Briefly, mRNA was isolated using the RNeasy^{®} Micro Kit (Qiagen, Hilden, Germany) following manufacturer's instructions. The sequencing library was prepared using 25 ng of total RNA by a Universal Plus mRNA-Seq NuGEN (0508, 9133, 9134, Tekan, Leek, The Netherlands). Single-end mRNA-seq was performed in the Illumina platform HiSeq2500. The dataset is available at www.shiny.Ivbrg.barcelona/Isec_PH. Venn diagrams were performed using the Venny2.1 free online software (by Juan Carlos Oliveros, BioinfoGP, CNB-CSIC). Canonical pathways analysis was performed using the Ingenuity Pathways Analysis software from Qiagen (content version: 49932394). Transcriptomics data from LSECs isolated from preclinical models of aCLD (CCl₄; thioacetamide, TAA; and common bile duct ligation, cBDL) and alcohol associated cirrhotic patients were obtained from Manicardi, N. and colleagues (Manicardi, N. *et a*/*.,* 2021).

### Gene Expression Analysis

Total RNA was extracted and purified with TRItidy G^{™} (A4051,0200, Panreac, Castellar del Vallès, Spain) and TRIzol:chloroform for liver tissues or using Qiazol lysis reagent and RNeasy^{®} Micro Kit (Qiagen) for primary cells according to the manufacturer instructions. RNA yield was quantified by Nanodrop ND-1000 spectrophotometer (NanoDrop Technologies, Wilmington, DE, USA). A total amount of 0.5 µg (tissue) and 0.15 µg (cells) of total RNA was reverse transcribed using a High-Capacity cDNA Reverse Transcription Kit (Applied Biosystems) in a thermal cycler (Eppendorf AG 22331, Hamburg, Germany), and qPCR was performed in a 7900HT Fast Real-Time PCR System (Thermofisher), using the TaqMan Universal PCR Master Mix (Applied Biosystems).

RNA expression levels were normalized following the 2-ΔΔCt method, with beta actin (Actb) or glyceraldehyde-3-phosphate dehydrogenase (Gapdh) as housekeeping genes. The following commercial Thermofisher TaqMan^{™} gene expression assay (FAM) probes were used: chromobox 7 (*CBX7,* Rn01506264_m1), Fc-gamma receptor IIb (*CD32b,* Rn00598391 m1), stabilin 2 (*Stab2,* Rn01503539_m1), *Gapdh* (Rn01775763_g1), hsamiR-181a-5p (000480) and U6 snRNA (001973). For human: *CBX7* (Hs00545603_m1) and Actb (Hs99999903_m1).

### Patients

Three different cohorts of patients from the Hospital Clinic of Barcelona (Spain) were used for this study; two including HVPG measurement and liver biopsy collected with an 18-G Tru-cut needle during PP measurement (discovery and validation cohort) and one including HVPG and blood sample collected via systemic venous access just before PP measurement (biomarker cohort).

The discovery cohort (31 subjects) was a retrospective one consisting in 12 healthy volunteers and 19 portal hypertensive cirrhotic patients due to HCV infection with similar ages and with the majority of patients with a Child Pug A grade (n= 16). Among the 19 portal hypertensive patients: 6 patients had a HVPG< 10 mmHg, 5 patients had 10<HVPG<12, and 8 patients had a HPVG> 12 mmHg.

The validation cohort was prospectively collected and comprised 11 subjects without PH (5 healthy volunteers, 5 patients with portal cavernomatosis and 1 patient with unspecific vascular aberrations), 41 portal hypertensive cirrhotic patients of HCV (n= 16) and OH (n= 25) aetiologies, and 14 HCV cirrhotic patients 12 months after receiving antiviral treatment:

**TABLE 1**

| | ***Group*** | ***N*** | ***HVPG (mmHg)*** |
|---|---|---|---|
| *Discovery cohort* | healthy | 12 | 4 ± 0.8 |
| | CLD | 19 | 12 ± 4.5 |
| *Validation cohort* | healthy | 11 | 3 ± 1.3 |
| | CLD | 41 | 15.6 ± 7.6 |
| | HCV + antiviral | 14 | 10.2 ± 3.6 |

The biomarker cohort was a prospective one with 18 normotensive healthy volunteers and 46 patients with CLD-derived portal hypertension.

Patients included in all cohorts underwent HVPG measurement due to clinical recommendation.

The protocol of this study was reviewed and approved by the Ethical and Clinical Investigation Committee of the Hospital Clinic of Barcelona and was in according with the Helsinki Declaration of 1975, as revised in 1983. Written informed consent was obtained from each patient.

### HVPG measurement

HVPG measurement was performed at the Barcelona Hepatic Hemodynamic Unit as described (Bosch, J. *et al.,* 2008). In short, a 7F balloon-tipped catheter ("Fogarty" Edwards Lifesciences LLC, CA) was guided into the main right or middle hepatic vein for measurements of wedged and free hepatic venous pressures. The HVPG resulted from the difference between both measurements.

All measurements were taken by triplicate and averaged to obtain the baseline HVPG. Permanent tracings were obtained in each case in a multichannel recorder (GE Healthcare, Milwaukee, WI).

### Plasma collection

For the biomarker cohort, blood was drawn from the subjects forming each one of the above cohorts, collected in BD Vacutainer^{®} K2 EDTA tubes (KFK286, Becton Dickinson), immediately kept at 4°C, centrifuged at 1300g for 10min at 4°C to obtain the plasma, and further centrifuged at 3000g for 15 min at 4°C to remove contaminating platelets. Plasma was aliquoted and stored in the IDIBAPS Biobank at -80°C following the internal agreement policy until used.

### Enzyme linked immunosorbent assay (ELISA)

Levels of E-cadherin (ECAD) and serine protease inhibitor Kazal-type 1 (SPINK1) in plasma was measured by ELISA following manufacturer's instructions. ECAD (ab233611) kits were purchased from Abcam (Cambridge, United Kingdom) and SPINK1 (DY7496-05) kits were purchased from R&D Systems (Abingdon, United Kingdom).

### Statistical analyses

Data are presented as mean ± standard deviation (SD) based on at least three independent experiments. Statistical analysis was performed using One-Way ANOVA or Student's t-test for parametric variables or Mann-Whitney U test for non-parametric tests. The correlations between *CBX7* expression and HVPG were calculated by Chi-squared test and Spearman's rank correlation. Differences were considered statistically significant for a p-value <0.05. Areas under the receiver operating characteristic (AUROC) curve and cut off values for the best specificity and sensitivity established, positive predictive value (PPV) and negative predictive value (NPV) were calculated. Statistical analyses were performed using GraphPad Prism 8.0.2 (GraphPad Software, Inc; San Diego, CA, USA) or SPSS (IBM, Chicago, IL, USA) software.

### Results

### Pathological hydrodynamic pressure induces LSECs dysfunction

The present inventors performed, a bulk RNA sequencing of LSECs cultured under physiological (1 mmHg) or pathological (12 mmHg) pressure in order to elucidate whether hydrodynamic pressure had a pathological effect on LSECs.

Transcriptomic analysis revealed 206 deregulated genes (197 downregulated and 9 upregulated) with a fold change ≥ 3 and p-value < 0.05 in response to pathological pressure. Interestingly, the inventors confirmed a global detrimental impact in LSEC homeostatic functions in view of the differentially expressed genes (DEG) identified, implied in different molecular processes categories, involved in LSECs metabolism, cell death and survival, proliferation, angiogenesis/remodelling, and inflammation.

This is the first time that it is reported the correlation between hydrodynamic pressure and LSEC dysfunction.

Table 2 shows the top 50 de-regulated genes in response to pathological hydrodynamic pressure.

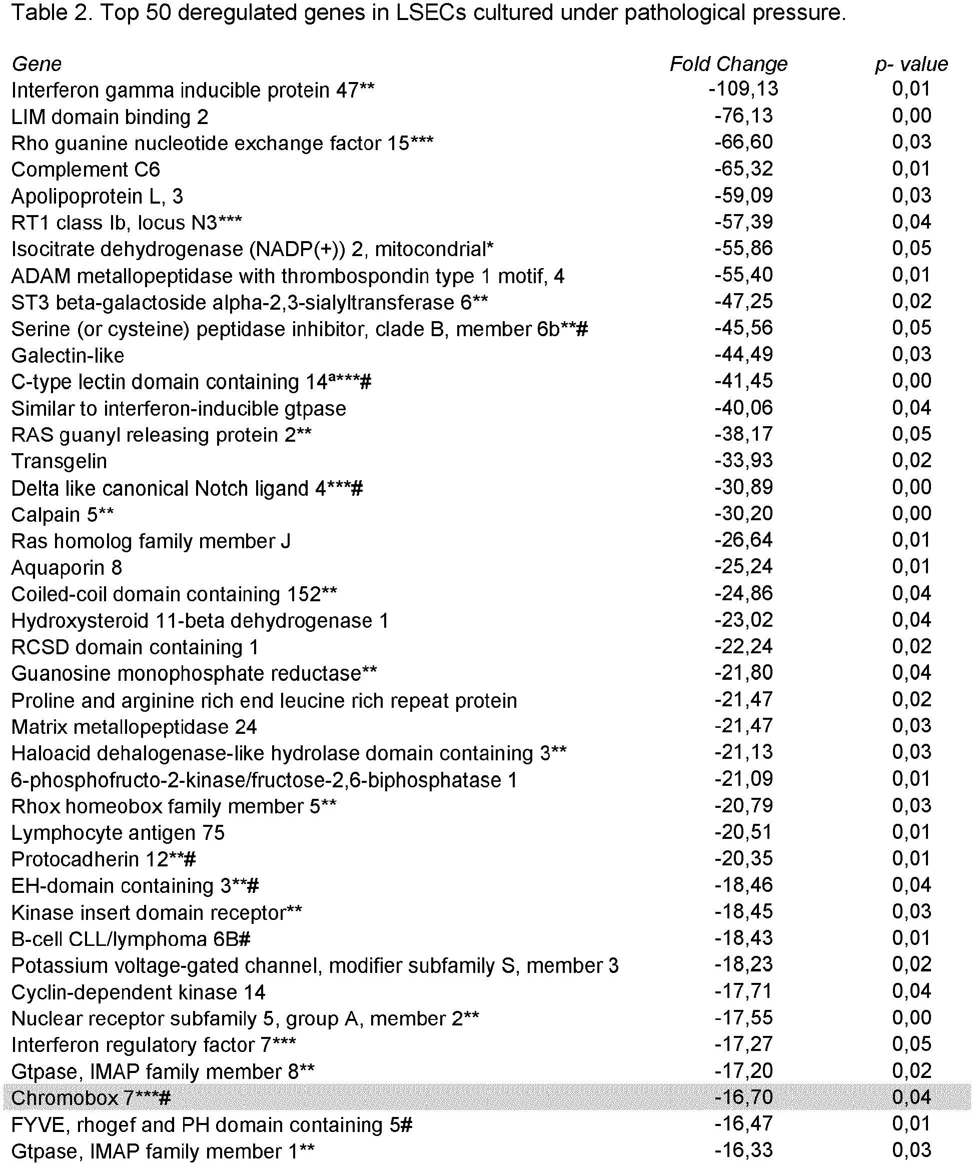

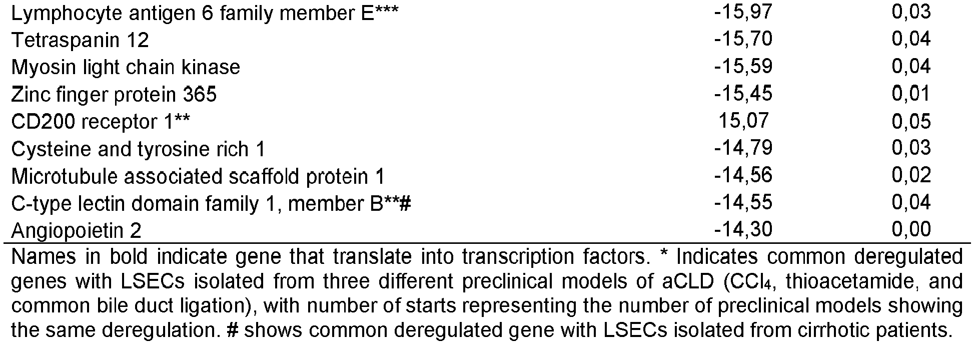

These are pressure-specific DEG which can provide information of LSEC dysfunction due to hydrodynamic pressure.

As increased pressure is part of the complex and multifactorial pathophysiology of aCLD, the inventors then compared the pressure specific DEG identified as explained above, with those reported as differentially expressed in LSECs isolated from 3 different preclinical models of aCLD: chronic CCl₄, chronic TAA and cBDL (Manicardi N. et al, 2021). Additionally, pressure-specific DEG were also compared to those from primary LSECs isolated from cirrhotic patients (hLSEC).

Comparison analysis between these groups showed that Chromobox 7 (*CBX7*) was the only gene with transcription regulation activity that was also downregulated in LSECs isolated from the three preclinical models of aCLD and in LSECs isolated from cirrhotic patients being the most promising candidate for assessing pressure-derived changes in LSECs.

### CBX7 downregulation correlates with HVPG in liver biopsies from aCLD patients

To investigate the potential role of CBX7 as a pressure-derived biomarker, the present inventors interrogated its expression in liver biopsies from two different cohorts of aCLD patients.

The discovery cohort included an array expression from healthy (n = 12) and HCV-cirrhotic patients (n=19) with similar ages, and with the majority of patients with a Child Pugh A grade (n=16). Moreover, 6 patients had an HVPG < 10 mmHg, 5 patients an HVPG between 10 and 12 mmHg, and 8 patients showed an HPVG over 12 mmHg.

Liver biopsies from the validation cohort included a higher number of patients with alcohol associated CLD (OH) (n = 25) and HCV (n = 16) aetiologies and were analyzed by qPCR. Patients mean age was 58.0 ± 7.5 years for the aCLD group and 56.1 ± 16.4 for the healthy group, both with a similar gender distribution. Child Pugh scores were also evenly distributed, with 16, 15 and 9 patients with scores of A, B and C, respectively. Regarding HVPG, 7 patients had a measurement below 10 mmHg, 8 patients between 10 and 12 mmHg, and 21 patients above 12 mmHg.

*CBX7* gene expression was analyzed in both cohorts comparing healthy volunteers (HVPG < 5 mmHg) against patients with PH (HVPG ≥ 5 mmHg).

It was found that CBX7 was significantly downregulated in patients with PH in both cohorts, as summarized in Table 3.

Interestingly, CBX7 downregulation was aetiology independent (and significantly correlated with HVPG . AUROC curves confidence interval (CI), cut-off values, sensibility, and specificity as well as PPV and NPV for the exploratory and validation cohorts are shown in Table 4 below.

AUROC values for the discovery and validation cohorts showed excellent diagnostic ability, being both above 0.965 with a sensitivity of 100% and specificities of at least 75% and 90% respectively. Aetiology-specific analysis in the validation cohort showed a better predictive power for HCV patients (AUROC: 0.994 vs 0.945 and specificity: 93.7 vs 88) compared to those from OH aetiology. Nevertheless, in both populations, CBX7 expression was highly sensible (100%) in detecting patients with PH. *CBX7* gene expression was also assessed in a small subgroup of HCV patients after 12 months of antiviral therapy treatment and its expression was significantly higher than in non-treated HCV patients (+148% versus non-treated patients, p-value 0.0134). Thus suggesting that CBX7 reflects improvements in HVPG in response to therapy.

Collectively, these results suggest that *CBX7* is a pressure-sensitive factor in LSECs and provide the overarching rational to further dissect the mechanisms that command *CBX7* expression.

**Table 3**

| | ***Group*** | ***N*** | ***CBX7 expression*** | ***p-value*** | ***Correlation with HVPG*** | ***p-value*** |
|---|---|---|---|---|---|---|
| *Discovery cohort* | NP | 12 | 1.00 ± 0.08 | < 0.0001 | R² = 0.439 | < 0.0001 |
| | PH | 19 | 0.75 ± 0.09 | | r = -0,6629 | |
| *Validation cohort (all)* | NP | 11 | 1.00 ± 0.19 | < 0.0001 | R² =0.259 | < 0.001 |
| | PH | 42 | 0.33 ± 0.25 | | r = -0,5096 | |
| *Validation cohort (OH)* | NP | 11 | 1.00 ± 0.19 | < 0.0001 | R² =0.317 | < 0.001 |
| | PH | 26 | 0.30 ± 0.27 | | r = -0,5632 | |
| *Validation cohort (HCV)* | NP | 11 | 1.00 ± 0.19 | < 0.0001 | R² =0.393 | < 0.001 |
| | PH | 16 | 0.39 ± 0.21 | | r = -0,6269 | |

**Table 4. Performance of CBX7 liver expression to predict portal hypertension in the discovery and validation cohorts.**

| | ***n (NP*/*PH)*** | ***AUROC (95% Cl)*** | ***Cut-off*** | ***Sens (%)*** | **Spec *(%)*** | ***PPV*** (***%***) | ***NPV (%)*** |
|---|---|---|---|---|---|---|---|
| *Discovery cohort* | 12/19 | 0.978 (0.938 to 1.000) | 0.950 | 100 | 75 | 86.36 | 100 |
| *Validation cohort (HCV* + *OH)* | 11/41 | 0.965 (0.920 to 1.000) | 0.677 | 100 | 90 | 97.39 | 100 |
| *Validation cohort (HCV)* | 11/16 | 0.994 (0.976 to 1.000) | 0.677 | 100 | 93.7 | 95.9 | 100 |
| *Validation cohort (OH)* | 11/25 | 0.945 (0.875 to 1.000) | 0.648 | 100 | 88 | 94.98 | 100 |

| | | | | | | | |
|---|---|---|---|---|---|---|---|
| Values of area under the ROC curve (AUROC), the 95% confidence interval (CI), the cut-off value with better sensitivity (Sens) and specificity (Spec), positive predictive value (PPV) and negative predictive value (NPV) | | | | | | | |

### CBX7 downstream targets as non-invasive biomarkers for PH

From the above findings, the inventors made a further effort in an attempt to find non-invasive biomarkers for assessing NCSPH/CSPH related to CBX7-regulated circulating proteins.

Thus, ECAD and SPINK1 expression was analyzed in plasma from the biomarker cohort, which included ACLD patients from three different aetiologies (HCV, OH and metabolic-associated CLD) with similar age and gender distribution. Reference values were considered from those patients with NP (HVPG < 5 mmHg) for assessing PH and from patients with an HVPG < 10 mmHg when assessing CSPH.

Plasmatic levels of ECAD and SPINK1 were significantly upregulated in patients with PH compared to patients with normal pressure (NP) (see Table 5 below).

AUROC curves to stratify patients between NP or PH were calculated for both markers alone or combined using the following formula: Y = 1 / 1 + exp(+4.503 - (0.06*ECAD(ng/ml))-(0.055*SPINK1(ng/ml)).A cut-off value of 0.579 for the combination of ECAD+SPINK1 exhibited the best AUROC (0.911) compared to those for the individual markers (0.889 and 0.747 for ECAD and SPINK1, respectively). Test performance statistics for ECAD+SPINK1 showed a 91.3% sensitivity and 83.3% specificity (Table 6 below).

When assessing CSPH, ECAD expression was significantly upregulated in patients with CSPH. AUROC curves for assessing patients below and above 10 mmHg was calculated for both markers and their combination using the formula as follows: Y = 1 / 1 + exp(+1.116 - (0.020*ECAD(ng/ml))-(0.013*SPINK1(ng/ml)).

The combination of ECAD+SPINK1 showed the best predictive power; when using a cut off of 0.647 test sensitivity was 91.4% and specificity reached 63.6%. Table 7 summarizes the results.

Based on these findings, it is proposed the determination of the plasmatic levels of ECAD and/or SPINK1, two of CBX7 targets, as non-invasive biomarkers for PH onset and CSPH monitoring in aCLD patients.

**Table 5**

| Groups | **N** | ***ECAD (ng*/*mL)*** | ***ECAD Fold* Change vs *NP (%)*** | ***SPINK1 (ng*/*mL)*** | ***SPINK1 Fold Changevs NP (%)*** |
|---|---|---|---|---|---|
| *NP* | 18 | 48.46 ± 21.83 | - | 16.71 ± 6.36 | - |
| PH | 47 | 107.21 ± 53.17 | +121.23 | 42.35 ± 57.69 | +153.44 |
| CSPH | 36 | 115.41 ± 56.39 | +138.18 | 47.08 ± 64.77 | +181.75 |

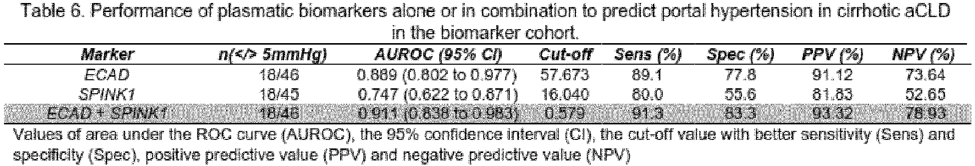

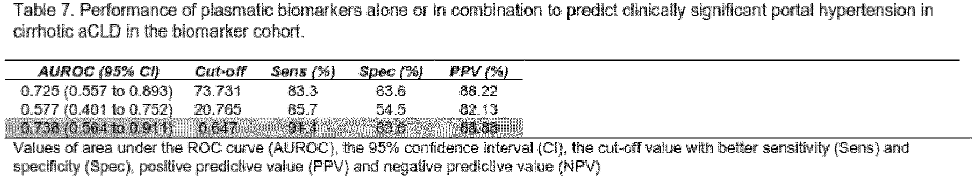

For reasons of completeness, various aspects of the invention are set out in the following numbered clauses:

### Clauses

**Clause 1.** A method of diagnosis of portal hypertension (PH) in a subject, the method comprising the steps of:
   (a) measuring the gene expression product of one or more CBX7 regulated protein selected from ECAD, SPINK1, FOS, FOSB, EGR1, HMGA1, p16/INK4a, ARF1, p15/INK4b and STEAP1 in an isolated sample from the subject;
   (b) comparing the amount with a reference value; and
   (c) diagnosing portal hypertension when the measured amount is higher than the reference value.
**Clause 2.** A method of diagnosis portal hypertension (PH) in a subject, the method comprising the steps of:
   (a) measuring the amount of the gene expression product of one or more CBX7 regulated protein selected from ECAD, SPINK1, FOS, FOSB, EGR1, HMGA1, p16/INK4a, ARF1, p15/INK4b and STEAP1 in an isolated fluid sample from the subject;
   (b) comparing the amount with a reference value; and
   (c) diagnosing the portal hypertension when the measured amount is higher than the reference value.
**Clause 3.** A method of prognosis PH, the method comprising measuring the amount of the gene expression product of one or more CBX7 regulated protein selected from ECAD, SPINK1, FOS, FOSB, EGR1, HMGA1, p16/INK4a, ARF1, p15/INK4b and STEAP1 in an isolated fluid sample from the subject, at least at two different temporal points; wherein if there is an increase in the amount of expression product, this indicates that there is a bad prognosis, particularly this is indicative that the subject is at risk of progressing to severe portal hypertension or decompensation.
**Clause 4.** A method for stratifying a subject as normal portal pressure, as suffering from non-clinically significant PH (NCSPH) or as suffering clinically significant PH (CSPH), the method comprising the steps of:
   (a) measuring the amount of expression gene product of one or more CBX7 regulated protein selected from ECAD, SPINK1, FOS, FOSB, EGR1, HMGA1, p16/INK4a, ARF1, p15/INK4b and STEAP1 in an isolated fluid test sample of the subject;
   (b) obtaining reference control values V1 and V2, wherein:
      V1 is calculated measuring the amount of the marker in a group of samples from subjects with a portal pressure equal or lower than 5 mmHg; and
      V2 is calculated measuring the amount of the marker in a group of samples from subjects with a portal pressure equal or lower than 10 mmHg, and higher than 5 mmHg;
   (c) comparing the measured amount of step (i) with the reference control values V1 and V2, wherein:
      if the measured value is equal or below V1, the subject is identified as "normal portal pressure";
      if the measured value is between V1 and V2, the subject is identified as suffering NCSPH; and
      if the measured value is equal or above V2, then the subject is identified as suffering CSPH.
**Clause 5.** A method of deciding or recommending whether to initiate a medical regimen of a subject suspicious of suffering PH, which method comprises the steps of:
   Step a:
      a.1) diagnosing the subject as suffering PH following the method as defined in clause 1; or, alternatively,
      a.2) stratifying the subject as NCSPH or CSPH following the method as defined in clause 3, and
   Step b: deciding or recommending to initiate the medical regimen if the subject is diagnosed as suffering PH or stratified as NCSPH or CSPH is recommended.
**Clause 6.** A method for determining the efficacy of a medical regimen in a patient already diagnosed of PH, the method comprising the steps of:
   (a) measuring the amount of the expression gene product of one or more CBX7 regulated protein selected from ECAD, SPINK1, FOS, FOSB, EGR1, HMGA1, p16/INK4a, ARF1, p15/INK4b and STEAP1 in an isolated fluid sample from the patient prior to the administration of the medical regimen;
   (b) measuring the amount of the expression gene product of the one or both markers in an isolated fluid sample from the patient once started the administration of the medical regimen; and
   (c) comparing the levels measured in steps (a) and (b), in such a way that if the amount measured in step (b) is lower than the amount measured in step (a), it is indicative that the medical regimen is effective in the treatment of the condition;
**Clause 7.** The method of any one of the preceding clauses, wherein the amount of the ECAD and/or SPINK1 markers, particularly of ECAD and SPINK1, is determined.
**Clause 8.** The method of any one of the preceding clauses, wherein the isolated fluid sample is selected from the group consisting of whole blood, plasma and serum.
**Clause 9.** A method of diagnosing or prognosing PH in a subject, which comprises measuring the amount of one or more of the markers provided in Table 2 in an isolated sample of the subject.
**Clause 10.** A method of stratifying a subject as a subject as normal portal pressure, as suffering from non-clinically significant PH (NCSPH) or as suffering clinically significant PH (CSPH), the method comprising the steps of:
   (a) measuring the amount of expression gene product of one or markers from Table 2 in an isolated test sample of the subject;
   (b) obtaining reference control values V1 and V2, wherein:
      V1 is calculated measuring the amount of the marker(s) in a group of samples from subjects with a portal pressure equal or lower than 5 mmHg; and
      V2 is calculated measuring the amount of the marker in a group of samples from subjects with a portal pressure equal or lower than 10 mmHg, and higher than 5 mmHg;
   (c) comparing the measured amount of step (i) with the reference control values V1 and V2, wherein:
      if the measured value is equal or above V1, the subject is identified as "normal portal pressure";
      if the measured value is between V1 and V2, the subject is identified as suffering NCSPH; and
      if the measured value is equal or below V2, then the subject is identified as suffering CSPH.
**Clause 11.** The method of any of the clauses 9-10, wherein the marker is CBX7.
**Clause 12** The method of any one of the clauses 9 and 11, wherein if the measured amount is lower than a reference control value, then the subject suffers from PH.
**Clause 13.** The method of any one of the clauses 9-12, wherein the isolated sample is a fluid sample, such as blood, serum or plasma, or an endothelial tissue such as liver tissue.
**Clause 14.** The method of any one of the preceding clauses, wherein the method further comprises
   i) measuring the level of one or more of the following markers: cholesterol; inflammatory biomarkers such as IL-1β, IL-IRa, Fas-R, VCAM1, TNF-β, HSP-70, IL-18, TLR9, lymphotoxin-β, glutamine, glutamine synthase, HSP-27, HSP-60, HSP-110, grpl70, hyaluronan, homocysteine, or angiotensin- II; and/or
   ii) further correlation with demographic and clinical laboratory parameters selected from the group consisting of age, model for end-stage liver diseases (MELD), Child-Pugh Score (CPS), platelets, alanine aminotransferase (ALT), aspartate aminotransferase (AST), platelet count, prothrombin time (PT/INR), liver stiffness, and at-risk alcohol use for indicating EGD to the patient or not indicating EGD for the patient.
**Clause 15.** The method of any one of the preceding clauses, wherein the expression gene product is protein.
**Clause 16.** The method of any one of the preceding clauses, wherein the measuring of the amount of the expression gene product in step (a) is performed by immunochemistry.
**Clause 17.** The method according to clause 16, wherein the level of expression of protein is determined using an antibody or a fragment thereof able to bind to the protein.
**Clause 18.** The method according to clause 17, wherein said antibody or fragment thereof forms part of a kit.
**Clause 19.** The method of clause 18, wherein the kit is an ELISA kit.
**Clause 20.** Use of ECAD alone or in combination with SPINK1 in the diagnosis and prognosis of PH; in the stratification of a subject as normal portal pressure, as suffering from non-clinically significant PH (NCSPH) or as suffering clinically significant PH (CSPH); in a method of deciding or recommending whether to initiate a medical regimen of a subject suspicious of suffering PH; or for determining the efficacy of a medical regimen in a patient already diagnosed of PH.
**Clause 21.** Use of means for performing any of the methods as defined in any one of the clauses 1 to 19.
**Clause 22.** The use of clause 21, wherein the means are antibodies or fragments thereof.
**Clause 23.** The use of any one of the clauses 21-22, wherein the means form part of a kit, such as an ELISA kit.
**Clause 24.** The method or use of any one of the preceding clauses, wherein the subject is one suffering from chronic liver disease, such as cirrhosis.

### Citation List

### Non Patent Literature

- Bosch, J. et al. " The management of portal hypertension: Rational basis, available treatments and future options", J. Hepatol., 2008, 48, 68-92;
- Burtis C. A. et al., 2008, Chapter 14, section "Statistical Treatment of Reference Values";
- Fernández-Iglesias, A. et al., "4 in 1: Antibody-free protocol for isolating the main hepatic cells from healthy and cirrhotic single rat livers", J. Cell. Mol. Med., 2019, 23, 877-886;
- Manicardi, N. et al., "Transcriptomic Profiling of the Liver Sinusoidal Endothelium during Cirrhosis Reveals Stage-Specific Secretory Signature", Cancers, 2021, 13(11), 2688-2704;
- Ortega-Ribera, M. et al., "Resemblance of the human liver sinusoid in a fluidic device with biomedical and pharmaceutical applications", Biotechnol. Bioeng., 2018, 115(10), 1-10; and
- Robin X. et al., 2009, "Bioinformatics for protein biomarker panel classification: What is needed to bring biomarker panels into in vitro diagnostics", Expert Review of Proteomics, 2009, 6 (6) p. 675-689.

## Claims

1. A method of diagnosis portal hypertension (PH) in a subject, the method comprising the steps of:
(a) measuring the amount of the gene expression product of one or both of the markers E-cadherin (ECAD) and SPINK1 in an isolated fluid sample from the subject;
(b) comparing the amount with a reference value; and
(c) diagnosing the portal hypertension when the measured amount is higher than the reference value.

2. A method of prognosis PH, the method comprising measuring the amount of the gene expression product of one or both of the markers ECAD and SPINK1 in an isolated fluid sample from the subject, at least at two different temporal points; wherein if there is an increase in the amount of expression product in the last temporal point with respect the previous one, this indicates that there is a bad prognosis, particularly this is indicative that the subject is in risk of progressing to severe portal hypertension or decompensation.

3. A method for stratifying a subject as normal portal pressure, as suffering from non-clinically significant PH (NCSPH) or as suffering clinically significant PH (CSPH), the method comprising the steps of:
(i) measuring the amount of expression gene product of one or both of the markers in an isolated fluid test sample of the subject;
(ii) obtaining reference control values V1 and V2, wherein:
V1 is calculated measuring the amount of the marker(s) in a group of samples from subjects with a portal vein pressure equal or lower than 5 mmHg; and
V2 is calculated measuring the amount of the marker in a group of samples from subjects with a portal pressure equal or lower than 10 mmHg, and higher than 5 mmHg;
(iii) comparing the measured amount of step (i) with the reference control values V1 and V2, wherein:
if the measured value is equal or below V1, the subject is identified as "normal portal pressure";
if the measured value is between V1 and V2, the subject is identified as suffering NCSPH; and
if the measured value is equal or above V2, then the subject is identified as suffering CSPH.

4. A method of deciding or recommending whether to initiate a medical regimen of a subject suspicious of suffering PH, which method comprises the steps of:
Step a:
a.1) diagnosing the subject as suffering PH following the method as defined in claim 1; or, alternatively,
a.2) prognosing the subject suffering PH following the method as defined in claim 1; or, alternatively,
a.3) stratifying the subject as NCSPH or CSPH following the method as defined in claim 3, and
Step b: deciding or recommending to initiate the medical regimen if the subject is diagnosed as suffering PH, or alternatively as having a bad prognosis, or alternatively as stratified as suffering from NCSPH or CSPH.

5. A method for determining the efficacy of a medical regimen in a patient already diagnosed of PH, the method comprising the steps of:
(a) measuring the amount of the expression gene product of one or both of the markers ECAD and SPINK1 in an isolated fluid sample from the patient prior to the administration of the medical regimen;
(b) measuring the amount of the expression gene product of the one or both markers in an isolated fluid sample from the patient once started the administration of the medical regimen; and
(c) comparing the levels measured in steps (a) and (b), in such a way that if the amount measured in step (b) is lower than the amount measured in step (a), it is indicative that the medical regimen is effective in the treatment of the condition.

6. The method of any one of the preceding claims, wherein the amount of both, the ECAD and SPINK1 markers, is determined.

7. The method of any one of the preceding claims, wherein the fluid test sample is selected from the group consisting of whole blood, plasma, serum, urine and saliva.

8. The method of any one of the preceding claims, wherein the expression gene product is protein.

9. The method of any one of the preceding claims, wherein the measuring of the amount of the expression gene product in step (a) is performed by immunochemistry.

10. The method according to claim 9, wherein the level of expression of protein is determined using an antibody or a fragment thereof able to bind to the protein.

11. The method according to claim 10, wherein said antibody or fragment thereof forms part of a kit, particularly an ELISA kit.

12. Use of ECAD alone or in combination with SPINK1 in the diagnosis and/or prognosis of PH; in the stratification of a subject as normal portal pressure, as suffering from non-clinically significant PH (NCSPH) or as suffering clinically significant PH (CSPH); in a method of deciding or recommending whether to initiate a medical regimen of a subject suspicious of suffering PH; or for determining the efficacy of a medical regimen in a patient already diagnosed of PH.

13. Use of means for performing any of the methods as defined in claims 1 to 11.

14. The use of claim 13, wherein the means are antibodies or fragments thereof.

15. The use of any one of the claims 13-14, wherein the means form part of a kit, particularly an ELISA kit.
